# FASCICULE DE BREVET EUROPEEN

(11) **EP 2 783 717 B1**
(45) Date de publication et mention de la délivrance du brevet: **11.03.2020**
(21) Numéro de dépôt: 14161186.3
(22) Date de dépôt: 21.03.2014
(51) Int. Cl.: A61M 1/36, B01D 39/16, B01J 20/26, B01J 20/32

(54) **Unité de filtration des leucocytes à adhésion des plaquettes réduite**
Filtereinrichtung zur Entfernung von Leukozyten mit verringerter Plätchenadhäsion
Leucocyte filtration unit with reduced platelets adherence

(30) Priorité: 27.03.2013 FR 1352779
(43) Date de publication de la demande: 01.10.2014
(73) Titulaire: MACO PHARMA, 59420 Mouvaux (FR)
(72) Inventeur: Ducoroy, Laurent, 59800 Lille (FR); Bessy, Emilie, 83120 Sainte Maxime (FR); Henard, Gregory, 59000 Lille (FR); Hupin, Christophe, 33770 Salles (FR); Dakhli, Sonia, 33000 Bordeaux (FR); Fouchet, Laurent, 33710 Prignac et Marcamps (FR)
(74) Mandataire: Sayettat, Julien Christian

(56) Documents cités:
- EP-A1- 1 336 417
- EP-A1- 1 452 193
- EP-A1- 1 553 113
- EP-A1- 2 075 018
- FR-A1- 2 892 949
- JP-A- H0 725 776
- JP-A- 2003 164 521
- JP-A- 2006 035 090
- JP-A- 2008 079 890
- US-A1- 2006 207 937
- MATSUDA T ET AL: "Surface coating of hydrophilic-hydrophobic block co-polymers on a poly(acrylonitrile) haemodialyser reduces platelet adhesion and its transmembrane stimulation", BIOMATERIALS, ELSEVIER SCIENCE PUBLISHERS BV., BARKING, GB, vol. 15, no. 6, 1 mai 1994 (1994-05-01), pages 417-422, XP024142363, ISSN: 0142-9612, DOI: 10.1016/0142-9612(94)90219-4 [extrait le 1994-05-01]

## Description

L'invention concerne une unité de filtration destinée à permettre la déleucocytation sélective d'un fluide contenant des plaquettes sanguines, ainsi qu'un système à poches comprenant une telle unité.

Elle s'applique typiquement à la filtration du sang ou d'un composant sanguin contenant des plaquettes tel qu'un plasma riche en plaquettes (PRP), un concentré plaquettaire (CP) ou un pool de concentrés plaquettaires.

Le sang total est constitué de deux types de composants : les cellules sanguines comprenant les globules rouges, les leucocytes et les plaquettes, et le plasma, liquide jaune pâle dans lequel les cellules sanguines sont en suspension.

Actuellement, on ne transfuse aux patients que les composants sanguins nécessaires à leur état. Par exemple, on ne transfuse que des concentrés plaquettaires aux patients atteints de thrombocytopénie, c'est-à-dire dont la teneur en plaquettes dans le sang est réduite.

Il s'est avéré que les leucocytes ont des effets indésirables très importants, ce qui a conduit à chercher à les éliminer des composants sanguins destinés à la transfusion. En effet, les leucocytes augmentent le risque de rejet immunitaire tel que la maladie du greffon contre l'hôte et favorisent la transmission d'agents infectieux. Il a aussi été démontré que les leucocytes affectaient de façon négative la conservation des plaquettes.

Pour éliminer les leucocytes des composants sanguins destinés à la transfusion, on connaît déjà des unités de filtration renfermant un milieu de déleucocytation. Dans de telles unités, le milieu de déleucocytation comprend une ou plusieurs membrane(s) et/ou une ou plusieurs couche(s) de non-tissé réalisée(s) en un matériau polymère et traitée(s) de façon à améliorer le taux de déleucocytation, la récupération des composants sanguins, le temps d'amorçage de la filtration et/ou la sélectivité de la filtration, par exemple en laissant passer les plaquettes.

Pour éliminer les leucocytes tout en laissant passer les plaquettes, plusieurs traitements de surface polymères de milieux de déleucocytation ont déjà été proposés.

Par exemple, le document US 4 936 998 décrit un milieu filtrant pour éliminer de façon sélective les leucocytes. Le milieu filtrant est composé de fibres revêtues d'un polymère contenant des groupes hydrophiles non ioniques et des groupes fonctionnels basiques contenant un azote. Un tel polymère est par exemple un copolymère de méthacrylate d'hydroxyéthyle et de méthacrylate de diéthylaminoéthyle.

Le document EP-1 230 940 décrit un filtre pour éliminer les leucocytes tout en laissant passer les plaquettes. Le filtre comprend un substrat revêtu, au moins à 70%, d'un polymère hydrophile synthétique ayant une masse moléculaire moyenne en masse comprise entre 300 000 et 3 000 000. Comme dans le document US 4 936 998, le polymère est un copolymère contenant des groupes hydrophiles non ioniques et des groupes fonctionnels basiques contenant un azote, obtenu par exemple par copolymérisation de méthacrylate d'hydroxyéthyle et de méthacrylate de diméthylaminoéthyle. Ce document indique en outre qu'avec un revêtement polymère ayant une masse moléculaire moyenne en masse en dessous de 300 000, le taux de passage des plaquettes diminue.

Le document JP 7-25776 propose un filtre pour éliminer de façon sélective les leucocytes comprenant sur sa surface un polymère doté d'une chaîne de polyéthylène glycol et de parties hydrophobes. Dans le document EP-1 452 193, il est cependant expliqué que ce polymère, du fait de la quantité élevée (59-74%) de chaîne d'éthylène glycol, présente des risques d'élution. Pour résoudre ce problème, Le document EP-1 452 193 propose d'utiliser un polymère obtenu à partir de : (i) un (méth)acrylate d'hydroxyalkyle, (ii) un monomère contenant des groupes azotes basiques et (iii) un monomère comprenant des chaînes d'oxyde d'éthylène contenant entre 2 et 9 répétitions d'oxyde d'éthylène. La masse moléculaire moyenne en masse du polymère est supérieure à 100 000 pour éviter les problèmes d'élution.

Une autre unité de filtration est décrite dans le document US-2006/0207937. Elle comprend un milieu de déleucocytation revêtu d'un polymère obtenu par réaction d'un monomère hydrophobe et d'un monomère hydrophile. Le milieu de déleucocytation possède une tension superficielle critique de mouillabilité CWST (critical wetting surface tension) comprise entre 50 et 80 dyn/cm. Par exemple, le polymère est un copolymère d'acétate de vinyle et de vinylpyrrolidone. Les polymères ont une masse moléculaire moyenne en masse (Mw) compris entre 10 000 et 200 000 g/mol.

Enfin, il a également été proposé dans la demande WO-2007/054638 d'enduire un milieu de déleucocytation d'un polymère linéaire de type Poloxamer® ayant une masse molaire comprise entre 2 000 et 18 000 g/mol. Cependant, ce type de revêtement polymère pose des problèmes d'élution.

Le document EP 1553113 divulgue un support dont la surface est revêtue d'un polymère pouvant être utilisé dans une unité de filtration destinée à permettre la déleucocytation sélective du sang. Le polymère présente des propriétés d'élution faible et permet de réduire l'adsorption des plaquettes.

La demanderesse a développé un nouveau traitement de surface à base d'un polymère insoluble dans l'eau et permettant la déleucocytation sélective d'un composant sanguin contenant des plaquettes. Contrairement à l'enseignement de l'art antérieur, le polymère de l'invention, résiste à l'élution et à la stérilisation vapeur, tout en permettant de retenir les leucocytes et de laisser passer les plaquettes.

A cet effet, l'invention propose une unité de filtration destinée à permettre la déleucocytation sélective d'un fluide contenant des plaquettes sanguines comprenant une enveloppe extérieure munie d'au moins un orifice d'entrée et d'au moins un orifice de sortie, l'enveloppe renfermant un élément poreux interposé entre lesdits orifices, ledit élément poreux renfermant un milieu de déleucocytation enduit d'un polymère, ledit polymère comportant une chaîne principale hydrophobe et une chaîne pendante hydrophile de poly(oxyde d'éthylène), ledit polymère possédant une masse molaire moyenne en masse (Mw) comprise entre 1 000 et 20 000 g/mol, la chaîne de poly(oxyde d'éthylène) comprenant entre 9 et 50 motifs d'oxyde d'éthylène et le pourcentage en masse de ladite chaîne de poly(oxyde d'éthylène) étant inférieur à 50% de la masse molaire moyenne en masse du polymère.

D'autres objets et avantages apparaîtront au cours de la description qui suit.
La figure 1 représente de façon schématique une vue en perspective d'une unité de filtration selon l'invention.
La figure 2 représente de façon schématique une vue en coupe de l'unité de filtration de la figure 1.
La figure 3 représente de façon schématique un équipement permettant de réaliser l'enduction d'un milieu de déleucocytation selon le principe de foulardage séchage.
La figure 4 représente de façon schématique un système à poches comprenant une unité de filtration selon l'invention.

L'invention propose une unité de filtration destinée à permettre la déleucocytation sélective d'un fluide contenant des plaquettes sanguines.

Un fluide contenant des plaquettes est par exemple un sang total, un plasma riche en plaquettes (PRP), un concentré plaquettaire (CP) ou un buffy-coat aussi appelé couche leuco-plaquettaire.

Notamment, l'unité de filtration est destinée à filtrer un pool de buffy-coats obtenu après réunion de deux à huit unités de buffy-coats issues d'un don de sang total.

En particulier, un pool de cinq buffy-coats est additionné d'une solution de conservation des plaquettes comme la solution SSP+ (Maco Pharma, France) avant d'être filtré.

L'unité de filtration permet une déleucocytation sélective, c'est-à-dire qu'elle est capable de retenir les leucocytes tout en laissant passer les plaquettes.

Notamment, l'unité de filtration est apte à obtenir un nombre de leucocytes dans le fluide filtré qui est inférieur à 1.10⁶ et à laisser passer au moins 80 % des plaquettes sanguines. En particulier, le fluide filtré au travers de l'unité de filtration comprend moins de 0,6.10¹¹ plaquettes pour 40 ml de fluide filtré, moins de 1.10⁵ leucocytes et le taux de récupération des plaquettes est d'au moins 90%.

Lorsque le fluide est un concentré plaquettaire, le nombre de plaquettes dans le concentré de plaquettes après filtration est d'au moins 2.10¹¹ plaquettes.

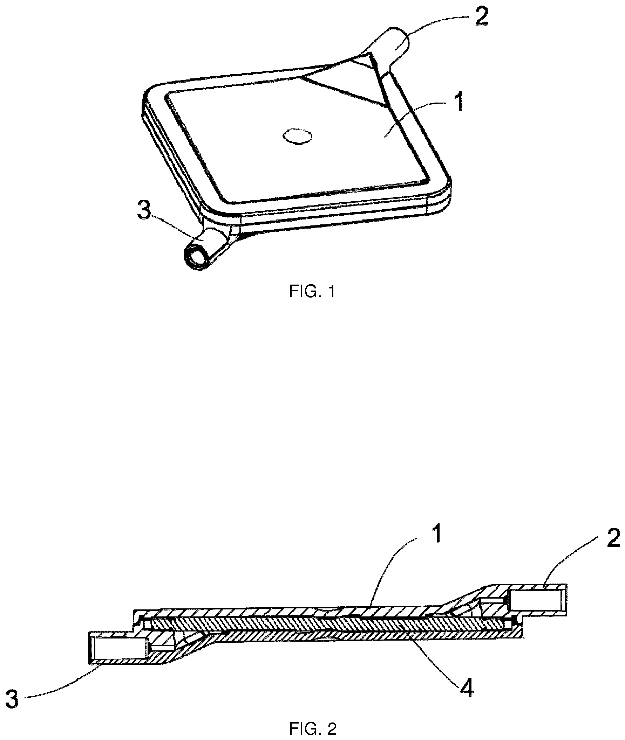

Comme représenté sur les figures 1 et 2, l'unité de filtration 1 comprend une enveloppe extérieure munie d'au moins un orifice d'entrée 2 et d'au moins un orifice de sortie 3, l'enveloppe renfermant un élément poreux 4 interposé entre lesdits orifices 2,3, ledit élément poreux 4 renfermant un milieu de déleucocytation enduit d'un polymère.

L'enveloppe extérieure de l'unité de filtration est souple, rigide ou semi-rigide. L'unité de filtration est avantageusement symétrique, de sorte à pouvoir être montée dans n'importe quel sens dans un système à poches, sans affecter les performances de filtration.

Le milieu de déleucocytation enduit d'un polymère est apte à retenir les leucocytes par adsorption et/ou par filtration des leucocytes présents dans le fluide contenant les plaquettes. Le milieu de déleucocytation enduit empêche également les plaquettes d'adhérer à sa surface.

Dans un milieu de déleucocytation, les leucocytes sont retenus par l'un et/ou l'autre des mécanismes suivants. Le premier mécanisme est l'adsorption des leucocytes à la surface du milieu. Les leucocytes sont adsorbés sur des surfaces cationiques et moyennement hydrophobes. La qualité de l'adsorption dépend également de la surface de contact disponible pour l'adsorption des leucocytes. Le deuxième mécanisme est le tamisage et dépend principalement du diamètre des pores du milieu de déleucocytation.

D'autre part, pour une filtration sélective, la surface du milieu de déleucocytation doit être est suffisamment hydrophile pour que les plaquettes n'adhèrent pas à sa surface.

Le milieu de déleucocytation de l'unité de filtration de l'invention est enduit d'un polymère, ledit polymère comportant une chaîne principale hydrophobe et une chaîne pendante hydrophile de poly(oxyde d'éthylène). Ce polymère est aussi appelé polymère d'enduction.

Ce polymère est un polymère en peigne, constitué d'une chaîne principale hydrophobe présentant au moins un point de ramification qui est le point de départ de la chaîne latérale linéaire hydrophile.

La chaîne hydrophobe contribue à l'adsorption des leucocytes ainsi qu'au maintient du polymère à la surface du milieu de déleucocytation. La chaîne hydrophile rend le milieu de déleucocytation suffisamment hydrophile pour ne pas faire adhérer les plaquettes.

Selon l'invention, le polymère possède une masse molaire moyenne en masse (Mw) comprise entre 1 000 et 20 000 g/mol, en particulier inférieure à 10 000 g/mol et notamment comprise entre 1 000 et 5 000 g/mol.

La masse molaire moyenne en masse est déterminée par exemple par chromatographie d'exclusion stérique dans le tétrahydrofurane avec un étalonnage polystyrène.

De plus, la chaîne de poly(oxyde d'éthylène) comprend entre 9 et 50 motifs d'oxyde d'éthylène et le pourcentage en masse de ladite chaîne de poly(oxyde d'éthylène) est inférieur à 50% de la masse molaire moyenne en masse du polymère.

La chaîne de poly(oxyde d'éthylène) permet d'augmenter le passage des plaquettes. Les chaînes courtes de poly(oxyde d'éthylène) contenant moins de 9 motifs d'oxyde d'éthylène ne permettent pas d'atteindre un caractère hydrophile suffisant lorsque le pourcentage en masse de ladite chaîne de poly(oxyde d'éthylène) est inférieur à 50% de la masse molaire moyenne en masse du polymère. Mais, les chaînes longues de poly(oxyde d'éthylène) contenant plus de 100 motifs d'oxyde d'éthylène forment une pelote statistique qui possède une capacité de répulsion stérique. Cette dernière réduit l'adsorption des cellules à la surface du milieu de déleucocytation, y compris celle des leucocytes.

Avantageusement, la chaîne de poly(oxyde d'éthylène) comprend entre 15 et 30 motifs d'oxyde d'éthylène. Cette longueur de chaîne pendante n'empêche pas les leucocytes d'adhérer au milieu de déleucocytation et permet de passiver la surface du milieu de déleucocytation pour ne pas retenir les plaquettes.

De plus, pour obtenir une filtration efficace en termes notamment de rétention des leucocytes, le polymère ne doit pas avoir un trop grand nombre de chaînes pendantes afin de ne pas perturber l'adsorption des leucocytes sur les parties hydrophobes du polymère. Ainsi, le pourcentage en masse de la chaîne de poly(oxyde d'éthylène) dans l'unité de filtration est inférieur à 50% de la masse molaire moyenne en masse du polymère.

Notamment le pourcentage en masse de la chaîne de poly(oxyde d'éthylène) est inférieur à 40% de la masse molaire moyenne en masse (Mw) du polymère. En particulier, il est compris entre 10 et 40%.

Comme le polymère est de faible masse molaire moyenne et comporte peu de chaînes pendantes hydrophiles, les chaînes hydrophobes du polymère restent facilement accessibles pour d'une part que le polymère adhère correctement au milieu de déleucocytation et d'autre part pour que les leucocytes s'adsorbent sur ces chaînes hydrophobes.

La composition massique du polymère est par exemple déterminée par spectroscopie par résonance magnétique nucléaire (RMN) de ¹H.

Par exemple, dans le cas d'un polymère constitué d'une unité monomère de méthacrylate de poly(oxyde d'éthylène) et d'une unité monomère de méthacrylate de méthyle, la RMN du ¹H permet de calculer le rapport du méthacrylate de méthyle sur oxyde d'éthylène, par la détermination du rapport des intensités des bandes à 3,3 ppm caractéristiques des groupes O⁻CH₃ du méthacrylate de méthyle et à 3,6 ppm caractéristique des groupes O⁻CH₂ de l'oxyde d'éthylène. Connaissant le nombre de motifs moyens d'oxyde d'éthylène dans une unité de méthacrylate de poly(oxyde d'éthylène), il est alors facile de calculer à la composition molaire en méthacrylate de poly(oxyde d'éthylène) et méthacrylate de méthyle. La connaissance des masses molaires de ces deux unités monomères permet de déterminer la composition massique du polymère.

L'indice de polymolécularité du polymère est compris entre 1 et 3, en particulier entre 1 et 2,5. L'indice de polymolécularité est le rapport de la masse molaire moyenne en masse (Mw) sur la masse molaire moyenne du polymère en nombre (Mn). Cet indice permet de caractériser globalement la dispersité des masses molaires d'un polymère. Un indice proche de 1 indique que toutes les chaînes molaires d'un polymère sont de même longueur.

Comme la masse molaire moyenne en masse, la masse molaire moyenne en nombre est déterminée par exemple par chromatographie d'exclusion stérique dans le tétrahydrofurane avec un étalonnage polystyrène.

Selon une réalisation de l'invention, la chaîne hydrophobe du polymère d'enduction dérive d'un monomère hydrophobe. Des exemples de monomères hydrophobes sont le styrène et les composés styréniques, les composés vinyliques tels que l'acétate de vinyle, les dérivés acrylates ou méthacrylates et les dérivés d'acrylamides. Par exemple, le monomère hydrophobe est un acrylate ou méthacrylate d'alkyle, tel que le méthacrylate de méthyle.

La chaîne pendante hydrophile dérive d'un macromonomère de poly(oxyde d'éthylène).

Les macromonomères sont des polymères ou oligomères ayant une extrémité réactive, par exemple un groupement fonctionnel polymérisable, qui lui permet de réagir comme un monomère.

Le groupement fonctionnel polymérisable peut être choisi parmi les groupes (méth)acryliques, vinyliques ou allyliques.

Avantageusement, le macromonomère de poly(oxyde d'éthylène) est un méthacrylate de poly(oxyde d'éthylène), le nombre de motifs d'oxyde d'éthylène dans la chaîne de poly(oxyde d'éthylène) étant compris entre 9 et 50.

En particulier, le polymère est un copolymère d'un monomère hydrophobe et d'un macromonomère hydrophile de poly(oxyde d'éthylène). Par exemple le copolymère peut être obtenu par copolymérisation d'un monomère de méthacrylate de méthyle et d'un monomère de méthacrylate de pol(oxyde d'éthylène).

Le polymère d'enduction ainsi obtenu est avantageusement insoluble dans l'eau, soluble dans un solvant alcoolique ou cétonique et résistant à la stérilisation vapeur.

Pour enduire le milieu de déleucocytation du polymère d'enduction, on prépare d'abord une solution d'imprégnation constituée du polymère d'enduction en tant que soluté et d'un liquide organique en tant que solvant.

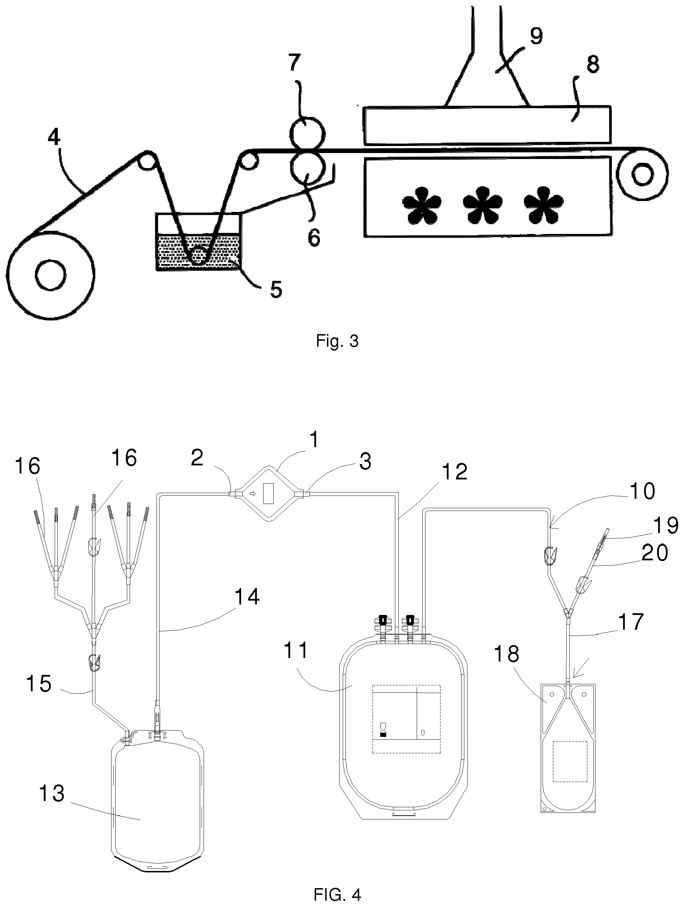

La solution d'imprégnation contient une faible concentration de polymère d'enduction. Notamment, la quantité de polymère dissous dans le solvant est comprise entre 5 et 50 g/L, en particulier comprise 10 et 20 g/L.
Les solvants d'imprégnation sont notamment un solvant alcoolique tel que le méthanol ou l'éthanol, ou un solvant cétonique tel que l'acétone ou la méthyléthylcétone.
Selon une réalisation, l'enduction est réalisée selon un principe de foulardage séchage, par exemple avec un équipement représenté sur la figure 3. Le milieu de déleucocytation 4 est trempé dans la solution d'imprégnation 5. Le surplus de solution enduite sur le milieu de déleucocytation est ensuite exprimé ou essoré en passant entre deux rouleaux 6,7 dont la pression est comprise entre 1 et 5 bars. Puis le milieu de déleucocytation 4 est convoyé dans un four 8 équipé d'une ventilation mécanique 9 afin de le sécher par évaporation du solvant. La vitesse, comprise entre 1 et 10 m/min, est régulée en fonction de la nature et de la quantité de solvant emportée par le milieu de déleucocytation. La quantité de polymère déposée sur le milieu de déleucocytation est comprise entre 10 et 50 mg/g de milieu de déleucocytation. Cette quantité est par exemple déterminée à l'aide d'un appareil d'extraction de Soxhlet puis par évaporation du solvant ou par chromatographie phase gaz. L'appareil de Soxhlet permet de faire l'extraction continue par solvant du polymère enduit sur le milieu de déleucocytation.

Le milieu de déleucocytation ainsi enduit présente une tension superficielle critique de mouillabilité CWST (critical wetting surface tension) comprise entre 65×10⁻³ et 90×10⁻³ N/m (65 et 90 dyn/cm), notamment entre 70×10⁻³ et 80×10⁻³ N/m (70 et 80 dyn/cm). Cette CWST est déterminée par la méthode décrite dans le document WO-8903717.

La CWST comprise entre 65×10⁻³ et 90×10⁻³ N/m (65 et 90 dyn/cm). caractérise la propriété bien hydrophile du milieu de déleucocytation enduit, c'est-à-dire qu'il peut être mouillé par le sang.
Comme vu plus haut, pour éviter l'adhésion des plaquettes sur le milieu de déleucocytation, la surface de celui-ci doit être hydrophile. Une CWST inférieure à 65×10⁻³ N/m (65 dyn/cm) ne prévient pas l'adhésion des plaquettes. Une CWST supérieure à 90×10⁻³ N/m (90 dyn/cm) empêcherait l'adsorption des leucocytes.
Idéalement, la CWST du milieu de déleucocytation est de l'ordre de 72×10⁻³ N/m (72 dyn/cm). En particulier, le caractère hydrophile d'un polymère comprenant une chaîne hydrophobe et une même chaîne pendante hydrophile de poly(oxyde d'éthylène) dépend non seulement du pourcentage en masse de la chaîne de poly(oxyde d'éthylène) dans le polymère mais aussi de la masse molaire moyenne du polymère.
Par exemple, un milieu de déleucocytation enduit d'un copolymère de méthacrylate de méthyle et de méthacrylate de poly(oxyde d'éthylène) de masse molaire 1 100 g/mol possède une CWST de 60×10⁻³ N/m (60 dyn/cm) lorsque le copolymère comprend 54% en masse de poly(oxyde d'éthylène) et possède une masse molaire moyenne en masse de 49 800 g/mol, alors qu'elle est de
72×10⁻³ N/m (72 dyn/cm) lorsque le polymère comprend 30% en masse de poly(oxyde d'éthylène) et possède une masse molaire moyenne en masse de 3 800 g/mol.

Selon une réalisation, le milieu de déleucocytation comprend des fibres de polyester, par exemple des fibres de polybutylène téréphtalate ou de polyéthylène téréphtalate.
Notamment, le milieu de déleucocytation est formé d'au moins une couche de non-tissé. En particulier, le milieu de déleucocytation comprend entre 5 et 40 couches de non-tissé, et plus particulièrement entre 10 et 20 couches de non-tissé. Par exemple, le diamètre des fibres de la ou des couches de non-tissé est compris entre 0,3 et 7 µm, avec une moyenne comprise entre 1 et 3 µm.

Pour retenir mécaniquement les leucocytes, le diamètre moyen des pores de la couche de non-tissé est avantageusement compris entre 3 et 15 µm, notamment entre 7 et 10 µm.

Avec une quantité de polymère déposée sur le milieu de déleucocytation comprise entre 10 et 50 mg/g de milieu de déleucocytation, le diamètre moyen des pores et le diamètre moyen des fibres de la couche de non-tissé avant et après enduction demeurent sensiblement les mêmes.

Selon une réalisation particulière, l'élément poreux de l'unité de filtration comprend en outre un pré-filtre et/ou post-filtre qui sont réalisés sous la forme d'au moins une couche de non-tissé et qui sont disposés respectivement côté amont et côté aval du milieu de déleucocytation.

Ces pré-filtres ou post-filtres ont notamment une taille de pores moyenne supérieure à celle du milieu de déleucocytation, par exemple comprise entre 25 et 50 µm.

Les pré-filtres ou post-filtres sont enduits ou non d'un polymère facilitant le passage des plaquettes.

Selon un autre aspect exemplifié sur la figure 4, l'invention concerne un système à poches 10 pour la déleucocytation d'un fluide contenant des plaquettes sanguines comprenant :
- une unité de filtration 1 selon le premier aspect de l'invention, et
- une poche de recueil du filtrat 11, ladite poche 11 étant reliée, par l'intermédiaire d'une tubulure 12, à un orifice de sortie 3 de l'unité de filtration 1.

En relation avec la figure 4, un système à poches particulier est décrit pour la préparation et la filtration d'un pool de buffy-coats.

Le système à poches 10 comprend une poche 13 de pool connectée ou destinée à être connectée par l'intermédiaire d'une première tubulure 14 à l'unité de filtration 1 de l'invention. L'unité de filtration 1 est connectée par l'intermédiaire d'une deuxième tubulure 12 à la poche 11 de recueil du filtrat.

Pour la préparation du pool de buffy-coats, la poche 13 de pool est en communication fluidique avec un ensemble de tubulures constitué d'une tubulure principale 15 et de tubulures secondaires 16 branchées sur la tubulure principale. Ces tubulures secondaires 16 sont destinées à être connectées de façon stérile à au moins deux poches contenant une unité de buffy-coat et éventuellement à une poche contenant une solution additive de conservation des plaquettes.

Selon une réalisation particulière, la poche 11 de recueil du filtrat est en communication fluidique par l'intermédiaire d'une troisième tubulure 17 à une poche satellite 18 destinée à recevoir l'air présent dans la poche 11 de recueil du filtrat et/ou au recueil d'un échantillon du fluide contenu dans la poche 11 de recueil du filtrat.

Afin de prélever des échantillons du fluide contenu dans la poche 11, le système à poches comprend une quatrième tubulure 20 branchée sur la troisième tubulure 17, ladite quatrième tubulure 20 étant pourvue à son extrémité d'un moyen de prélèvement 19. L'échantillon prélevé par ce moyen est utilisé pour la détection d'une contamination bactérienne (Système Bact/Alert®).

Les tubulures sont pourvues de clamp pour contrôler l'écoulement des fluides à l'intérieur du système à poches.

En relation avec le système à poches de la figure 4, on décrit maintenant une utilisation du système à poches 10.

Entre quatre et six poches contenant une unité de buffy-coat sont connectées de façon stérile, par exemple à l'aide d'un appareil de connexion stérile de type SCD (Terumo) aux tubulures secondaires 16 d'un système à poches 10. Une poche de solution de conservation de type SSP+ (Maco Pharma, France) est également connectée de façon stérile à l'une des tubulures secondaires 16.

Les buffy-coats s'écoulent dans la poche 13 de pool. Puis on transfère la solution de conservation vers la poche 13 de pool. L'ensemble de tubulures 15,16 est ensuite séparé de la poche 13 de pool par soudure.

Puis, la poche 13 de pool est centrifugée pour obtenir une couche sédimentaire de globules rouges et une couche de surnageant de concentré plaquettaire.

La poche 13 de pool contenant les couches de globules rouges et de concentré plaquettaire est ensuite pressée, par exemple à l'aide d'un dispositif de presse de type Macopress Smart (Maco Pharma, France) afin d'envoyer le concentré plaquettaire dans la poche 11 de recueil du filtrat via l'unité de filtration 1 de l'invention et d'obtenir un concentré de plaquettes déleucocyté.

### Exemple 1

### Préparation du polymère d'enduction

Un méthacrylate de poly(oxyde d'éthylène) (masse molaire : 475 g/mol ; nombre de motif d'oxyde d'éthylène = 9) a été solubilisé dans de l'éthanol avant d'être copolymérisé avec du méthacrylate de méthyle. Le copolymère (copolymère A) est lyophilisé et séché à 100°C sous vide pendant 4 heures.

Le copolymère A obtenu est non soluble dans l'eau. Sa masse molaire en masse est d'environ 7300 g/mol, son indice de polydispersité est de 2,4. Le polymère comprend 45% en masse de méthacrylate de poly(oxyde d'éthylène) et 55% en masse de méthacrylate de méthyle.

### Solution d'imprégnation

La solution d'imprégnation est préparée en solubilisant le polymère dans de l'éthanol afin d'obtenir une solution d'imprégnation comprenant 96% en masse d'éthanol et 4% en masse du copolymère.

### Enduction

Quinze couches de non-tissé de polybutylène téréphtalate (42 g/m², diamètre moyen de pores de 8,5 µm) ont été imprégnées avec la solution préparée ci-dessus et selon le principe du foulardage séchage décrit ci-dessus. La CWST des couches est de 72×10⁻³ N/m (72 dyn/cm). La quantité de polymère déposée est d'environ 20 mg de copolymère par gramme de non-tissé.

### Filtration

Une unité de filtration a été réalisée comportant un milieu de déleucocytation formée de quinze couches enduites du copolymère A.
Un concentré plaquettaire préparé à partir d'un pool de cinq buffy-coats est filtré au travers de l'unité de filtration.
La filtration a duré 4 minutes 50. Le volume de concentré plaquettaire après filtration est de 319 ml. Le concentré de plaquettes contient 4,45.10¹¹ plaquettes (soit un taux de récupération de 86 %) et 1,18.10⁵ globules blancs.

### Exemple 2

L'exemple 1 a été répété avec un copolymère B obtenu par copolymérisation d'un méthacrylate de poly(oxyde d'éthylène) de masse molaire 1100 g/mol et du méthacrylate de méthyle.
Le copolymère B obtenu est non soluble dans l'eau. Sa masse molaire en masse est d'environ 3800 g/mol, son indice de polydispersité est de 1,7. Le polymère comprend 30% en masse de méthacrylate de poly(oxyde d'éthylène) et 70% en masse de méthacrylate de méthyle.

Les résultats de filtration sont les suivants : La filtration a duré 5 minutes 12. Le volume de concentré plaquettaire après filtration est de 310 ml. Le concentré de plaquettes contient 3,5.10¹¹ plaquettes (soit un taux de récupération de 83 %) et 5,42.10⁴ globules blancs.

En comparant les résultats des exemples 1 et 2, on en déduit que des chaînes hydrophiles plus longues mais moins nombreuses, présentent une efficacité similaire pour la non-adhésion des plaquettes, mais permettent une meilleure adhésion des leucocytes.

### Exemple 3

L'exemple 1 a été répété en utilisant une unité de filtration comprenant un milieu de déleucocytation formé de quinze couches de non-tissé de polybutylène téréphtalate (52 g/m², diamètre moyen de pores de 10 µm), enduites avec la solution d'imprégnation de copolymère A de l'exemple 1.

Les résultats de filtration sont les suivants : La filtration a duré 69 secondes. Le volume de concentré plaquettaire après filtration est de 265 ml. Le concentré de plaquettes contient 2,59.10¹¹ plaquettes (soit un taux de récupération de 89 %) et 1,06.10⁵ globules blancs.

### Exemple comparatif

L'exemple 3 a été répété avec un copolymère C comprenant une chaîne hydrophile dérivé d'un méthacrylate de poly(oxyde d'éthylène) de masse molaire 1100 g/mol et d'une chaîne hydrophobe de poly(oxyde de propylène).

Le copolymère C obtenu est non soluble dans l'eau. Sa masse molaire en masse est d'environ 51 000 g/mol, son indice de polydispersité est de 1,5.

Les résultats de filtration sont les suivants : La filtration a duré 54 secondes. Le volume de concentré plaquettaire après filtration est de 178 ml. Le concentré de plaquettes contient 7,64.10¹⁰ plaquettes (soit un taux de récupération de 65 %) et 1,42.10⁴ globules blancs.

## Revendications

1. Unité de filtration (1) destinée à permettre la déleucocytation sélective d'un fluide contenant des plaquettes sanguines comprenant une enveloppe extérieure munie d'au moins un orifice d'entrée (2) et d'au moins un orifice de sortie (3), l'enveloppe renfermant un élément poreux (4) interposé entre lesdits orifices, ledit élément poreux renfermant un milieu de déleucocytation enduit d'un polymère, ledit polymère comportant une chaîne principale hydrophobe et une chaîne pendante hydrophile de poly(oxyde d'éthylène), **caractérisée en ce que** ledit polymère possède une masse molaire moyenne en masse (Mw) comprise entre 1 000 et 20 000 g/mol et **en ce que** la chaîne de poly(oxyde d'éthylène) comprend entre 9 et 50 motifs d'oxyde d'éthylène et le pourcentage en masse de ladite chaîne de poly(oxyde d'éthylène) étant inférieur à 50% de la masse molaire moyenne en masse du polymère.

2. Unité de filtration (1) selon la revendication 1, **caractérisée en ce que** la chaîne hydrophobe dérive d'un monomère hydrophobe choisi parmi les acrylates ou méthacrylates d'alkyle.

3. Unité de filtration (1) selon l'une des revendications 1 ou 2, **caractérisée en ce que** la chaîne pendante hydrophile dérive d'un macromonomère de poly(oxyde d'éthylène).

4. Unité de filtration (1) selon la revendication 3, **caractérisée en ce que** ledit macromonomère de poly(oxyde d'éthylène) est un méthacrylate de poly(oxyde d'éthylène).

5. Unité de filtration (1) selon l'une quelconque des revendications 1 à 4, **caractérisée en ce que** la chaîne de poly(oxyde d'éthylène) comprend entre 15 et 30 motifs d'oxyde d'éthylène.

6. Unité de filtration (1) selon l'une quelconque des revendications 1 à 5, **caractérisée en ce que** la masse molaire moyenne en masse dudit polymère est inférieure à 10 000 g/mol.

7. Unité de filtration (1) selon l'une quelconque des revendications 1 à 6, **caractérisée en ce que** le pourcentage en masse de la chaîne de poly(oxyde d'éthylène) est inférieur à 40% de la masse molaire moyenne en masse (Mw) du polymère.

8. Unité de filtration (1) selon l'une quelconque des revendications 1 à 7, **caractérisée en ce que** le polymère possède un indice de polymolécularité compris entre 1 et 3.

9. Unité de filtration (1) selon l'une quelconque des revendications 1 à 8, **caractérisée en ce que** la quantité de polymère déposée sur le milieu de déleucocytation est comprise entre 10 et 50 mg/g de milieu de déleucocytation.

10. Unité de filtration (1) selon l'une quelconque des revendications 1 à 9, **caractérisée en ce que** le milieu de déleucocytation présente une tension superficielle critique de mouillabilité CWST (critical wetting surface tension) comprise entre 65×10⁻³ et 90×10⁻³ N/m (65 et 90 dyn/cm).

11. Unité de filtration (1) selon l'une quelconque des revendications 1 à 10, **caractérisée en ce que** le milieu de déleucocytation comprend des fibres de polybutylène téréphtalate ou de polyéthylène téréphtalate.

12. Unité de filtration (1) selon l'une quelconque des revendications 1 à 11, **caractérisée en ce que** le milieu de déleucocytation est formé d'au moins une couche de non-tissé.

13. Unité de filtration (1) selon la revendication 12, **caractérisée en ce que** le diamètre moyen des pores de la couche de non-tissé est compris entre 3 et 15 µm.

14. Système à poches pour la déleucocytation d'un fluide contenant des plaquettes sanguines, **caractérisé en ce qu'**il comprend :
- une unité de filtration (1) selon l'une quelconque des revendications 1 à 13, et
- une poche (11) de recueil du filtrat, ladite poche étant reliée, par l'intermédiaire d'une tubulure (12), à un orifice de sortie (3) de l'unité de filtration (1).

## Patentansprüche

1. Filtrationseinheit (1), die dazu bestimmt ist, die selektive Deleukozytierung eines Blutplättchen enthaltenden Fluids zu ermöglichen, die eine Außenhülle umfasst, welche mit mindestens einer Einlassöffnung (2) und mindestens einer Auslassöffnung (3) ausgestattet ist, wobei die Hülle ein poröses Element (4) umschließt, das zwischen den Öffnungen eingefügt ist, wobei das poröse Element ein mit einem Polymer überzogenes Deleukozytierungsmedium umschließt, wobei das Polymer eine hydrophobe Hauptkette und eine hydrophile Seitenkette aus Poly(ethylenoxid) umfasst, **dadurch gekennzeichnet, dass** das Polymer eine gewichtsmittlere Molmasse (Mw) zwischen 1.000 und 20.000 g/mol besitzt und dadurch, dass die Poly(ethylenoxid)-Kette zwischen 9 und 50 Ethylenoxideinheiten umfasst und der Massenanteil der Poly(ethylenoxid)-Kette weniger als 50 % der gewichtsmittleren Molmasse des Polymers beträgt.

2. Filtrationseinheit (1) nach Anspruch 1, **dadurch gekennzeichnet, dass** die hydrophobe Kette von einem hydrophoben Monomer abgeleitet ist, das aus Alkylacrylaten oder -methacrylaten ausgewählt ist.

3. Filtrationseinheit (1) nach einem der Ansprüche 1 oder 2, **dadurch gekennzeichnet, dass** die hydrophile Seitenkette von einem Poly(ethylenoxid)-Makromonomer abgeleitet ist.

4. Filtrationseinheit (1) nach Anspruch 3, **dadurch gekennzeichnet, dass** das Poly(ethylenoxid)-Makromonomer ein Poly(ethylenoxid)-Methacrylat ist.

5. Filtrationseinheit (1) nach einem der Ansprüche 1 bis 4, **dadurch gekennzeichnet, dass** die Poly(ethylenoxid)-Kette zwischen 15 und 30 Ethylenoxideinheiten umfasst.

6. Filtrationseinheit (1) nach einem der Ansprüche 1 bis 5, **dadurch gekennzeichnet, dass** die gewichtsmittlere Molmasse des Polymers weniger als 10.000 g/mol beträgt.

7. Filtrationseinheit (1) nach einem der Ansprüche 1 bis 6, **dadurch gekennzeichnet, dass** der Massenanteil der Poly(ethylenoxid)-Kette weniger als 40 % der gewichtsmittleren Molmasse (Mw) des Polymers beträgt.

8. Filtrationseinheit (1) nach einem der Ansprüche 1 bis 7, **dadurch gekennzeichnet, dass** das Polymer einen Polymolekularitätsindex zwischen 1 und 3 besitzt.

9. Filtrationseinheit (1) nach einem der Ansprüche 1 bis 8, **dadurch gekennzeichnet, dass** die auf dem Deleukozytierungsmedium abgeschiedene Polymermenge zwischen 10 und 50 mg/g Deleukozytierungsmedium beträgt.

10. Filtrationseinheit (1) nach einem der Ansprüche 1 bis 9, **dadurch gekennzeichnet, dass** das Deleukozytierungsmedium eine kritische Benetzungsoberflächenspannung CWST (Critical Wetting Surface Tension) zwischen 65x10⁻³ und 90x10⁻³ N/m (65 und 90 dyn/cm) aufweist.

11. Filtrationseinheit (1) nach einem der Ansprüche 1 bis 10, **dadurch gekennzeichnet, dass** das Deleukozytierungsmedium Fasern aus Polybutylenterephthalat oder Polyethylenterephthalat umfasst.

12. Filtrationseinheit (1) nach einem der Ansprüche 1 bis 11, **dadurch gekennzeichnet, dass** das Deleukozytierungsmedium aus mindestens einer Vliesschicht gebildet ist.

13. Filtrationseinheit (1) nach Anspruch 12, **dadurch gekennzeichnet, dass** der mittlere Durchmesser der Poren der Vliesschicht zwischen 3 und 15 µm beträgt.

14. Beutelsystem zur Deleukozytierung eines Blutplättchen enthaltenden Fluids, **dadurch gekennzeichnet, dass** es umfasst:
- eine Filtrationseinheit (1) nach einem der Ansprüche 1 bis 13, und
- einen Beutel (11) zum Sammeln des Filtrats, wobei der Beutel mittels einer Schlauchleitung (12) an eine Auslassöffnung (3) der Filtrationseinheit (1) angeschlossen ist.

## Claims

1. Filtration unit (1) intended to allow for the selective leukodepletion of a fluid containing blood platelets comprising an outer envelope provided with at least one inlet orifice (2) and with at least one outlet orifice (3), with the envelope enclosing a porous element (4) inserted between said orifices, said porous element enclosing a leukodepletion medium coated with a polymer, said polymer including a main hydrophobic chain and a hydrophilic pendant chain of poly(ethylene oxide), **characterised in that** said polymer has a weight average molecular weight (Mw) comprised between 1,000 and 20,000 g/mol and **in that** the poly(ethylene oxide) chain comprises between 9 and 50 units of ethylene oxide and the weight percentage of said poly(ethylene oxide) chain being less than 50% of the weight average molecular weight of the polymer.

2. Filtration unit (1) according to claim 1, **characterised in that** the hydrophobic chain derives from a hydrophobic monomer chosen from alkyl acrylates or methacrylates.

3. Filtration unit (1) according to one of claims 1 or 2, **characterised in that** the hydrophilic pendant chain derives from a poly(ethylene oxide) macromonomer.

4. Filtration unit (1) according to claim 3, **characterised in that** said poly(ethylene oxide) macromonomer is a poly(ethylene oxide) methacrylate.

5. Filtration unit (1) according to any of claims 1 to 4, **characterised in that** the poly(ethylene oxide) chain comprises between 15 and 30 units of ethylene oxide.

6. Filtration unit (1) according to any of claims 1 to 5, **characterised in that** the weight average molecular weight of said polymer is less than 10,000 g/mol.

7. Filtration unit (1) according to any of claims 1 to 6, **characterised in that** the weight percentage of the poly(ethylene oxide) chain is less than 40% of the weight average molecular weight (Mw) of the polymer.

8. Filtration unit (1) according to any of claims 1 to 7, **characterised in that** the polymer has a polymolecularity index comprised between 1 and 3.

9. Filtration unit (1) according to any of claims 1 to 8, **characterised in that** the quantity of polymer placed on the leukodepletion medium is comprised between 10 and 50 mg/g of leukodepletion medium.

10. Filtration unit (1) according to any of claims 1 to 9, **characterised in that** the leukodepletion medium has a CWST (critical wetting surface tension) comprised between 65x10⁻³ and 90x10⁻³ N/m (65 and 90 dyn/cm) .

11. Filtration unit (1) according to any of claims 1 to 10, **characterised in that** the leukodepletion medium comprises polybutylene terephthalate or polyethylene terephthalate fibres.

12. Filtration unit (1) according to any of claims 1 to 11, **characterised in that** the leukodepletion medium is formed from at least one non-woven layer.

13. Filtration unit (1) according to claim 12, **characterised in that** the average diameter of the pores of the non-woven layer is comprised between 3 and 15 µm.

14. System with pouches for the leukodepletion of a fluid containing blood platelets, **characterised in that** it comprises:
- a filtration unit (1) according to any of claims 1 to 13, and
- a pouch (11) for collecting the filtrate, said pouch being connected, via a tubing (12), to an outlet orifice (3) of the filtration unit (1).
